# EUROPEAN PATENT APPLICATION

(11) **EP 0 893 115 A2**
(43) Date of publication of application: **27.01.1999**
(21) Application number: 98113135.2
(22) Date of filing: 15.07.1998
(51) Int. Cl.: A61F 13/56

(54) **Disposable diaper**

(30) Priority: 25.07.1997 JP 199602/97
(71) Applicant: YKK CORPORATION, Chiyoda-ku, Tokyo (JP)
(72) Inventor: Suenaga, Tomohiro, Ikoma-shi, Nara-ken (JP)
(74) Representative: Casalonga, Axel

(57) **Abstract**

In a disposable diaper (1), a pair of male surface fastener tapes (50) extends laterally in opposite directions from a rear flap (20) of a diaper body (1), each having a multiplicity of male engaging elements (52) on a substrate sheet (51). A part of outer surface of a front flap (10) of the diaper body (1) and/or a covering sheet (60) extending in a longitudinal direction from a front waist (12) of said front flap (10) is a non-woven cloth serving as a female surface fastener tape. In use, the covering sheet (60) is folded over the front part (10) so as to brought the non-woven cloth into engagement with the male engaging elements (52) of the male surface fastener tapes (50).

## Description

### BACKGROUND OF THE INVENTION

### 1. Field of the Invention:

The present invention relates to a disposable diaper, and more particularly to a disposable diaper which is low in cost of production and can be fastened compactly and stably in folded posture with adequate peeling strength and can be unfastened with a small zip sound.

### 2. Description of the Related Art:

In recent years application of disposable diapers whose chief materials are non-woven cloth or paper has been on the increase for either infants or aged persons. Among various diaper, paper diapers for infants or sick persons have been typical products. In these conventional diaper products, a diaper body is composed of outer and inner sheets and a liquid absorbing layer intermediate between the outer and inner sheets; in use, the diaper body is detachably attached to the user by a fastening member.

The general idea of these diapers includes potential applications in various arts such as a sweatband to be wound round a wrist or a head to soak up sweat especially during playing sports and also as a sanitary band to protect a wound on the skin. In the future, it is expected that their range of uses would be enlarged to cover even a medical-operation garment.

In the meantime, the demands of the users of disposable products as described in the above vary from good in liquid absorption, ventilation, leak-resistance and touch or texture, which are fundamental requirements to the products, to a simple structure and rational price, easy attaching and removing at any time except in use.

For example, in an absorbent disposable diaper disclosed in Japanese Patent Laid-Open Publication No. Hei 5-200069, in order to prevent the diaper from being inadvertently removed off the user's body and to facilitate attaching and removing of the diaper by the helper, two kinds of fastening member, i.e., male and female type surface fasteners and snap buttons are employed.

In the diaper disclosed in the above-mentioned publication, since the male and female surface fastener members cannot be unfastened until after the snap buttons are unfastened, it could reduce possibilities that the diaper might inadvertently removed off due to the movements of the infant. However, this known structure is so complex as to increase the number of manufacturing steps and hence the cost of production.

In the conventional diapers, including the abovementioned publication, it is common to employ male and female type surface fasteners as a fastening member, and to use a usual pile product woven or knitted of fibers the female surface fastener member. This is because it is easy to ensure adequate reaction with respect to opposite parallel stresses on the engaged surfaces and also adequate peeling strength corresponding to the stresses occurred when the male and female surface fastener members are peeled off each other. But the usual pile product woven or knitted of fibers are expensive, and accordingly the price of the disposable paper diaper would be high.

With the foregoing problems in view, the present inventors have proposed an improved disposable diaper by Japanese Patent Application No. Hei 8-10048.

In this proposed disposable diaper, a female surface fastener member is produced independently of a companion male surface fastener member, and then adhered to a diaper body. Therefore, its manufacturing process is substantially the same as that of other conventional diapers, thus causing an unsatisfied cost of production. Further, partly since the female surface fastener member is in the form of a sheet one of whose half parts (a first half) is adhered to the diaper body at a position off its front edge ( front waist), and partly since the remaining half part (a second half) of the sheet, which part is not adhered to the diaper body, is folded so as to cover the fastened portions of the surface fastener members, the second half of the female surface fastener member merely plays an auxiliary role to increase the engaging strength between the male and female surface fastener members. Accordingly, the peeling strength still relies on the engaging strength of the male and female surface fastener members.

### SUMMARY OF THE INVENTION

With the foregoing problems in mind, it is an object of the present invention to provide a disposable diaper which has a simple structure and is rational in manufacturing cost, which can be fastened with adequate engaging strength using high-productive male and female surface fastener members and can be simply attached to any user's body and removed off the body with a low zip sound as the male and female surface fastener members are peeled off each other, and which is also ecological.

According to a first aspect of the invention, the above object is accomplished by a disposable diaper comprising: a diaper body, a pair of male surface fastener tapes, and a covering sheet as follows. The diaper body has a front flap, a rear flap and a crotch. In the diaper body, at least a part of outer surface of the front flap is defined by a non-woven cloth which serves as a female surface fastener tape. The pair of male surface fastener tapes are extending laterally in opposite directions from the rear flap. Each of the male surface fastener tapes is composed of a substrate sheet and a multiplicity of male engaging elements standing on the substrate sheet. The covering sheet has a width substantially equal to the width of the diaper body, and is extending in a longitudinal direction from a front waist of the front flap. The covering sheet is foldable over the non-woven cloth.

According to a second aspect of the invention, the above object is accomplished in an alternative form by a disposable diaper comprising a diaper body, a pair of male surface fastener tapes, and a covering sheet as follows. The diaper body has a front flap, a rear flap and a crotch. The pair of male surface fastener tapes are extending laterally in opposite directions from the rear flap. Each of the male surface fastener tapes is composed of a substrate sheet and a multiplicity of male engaging elements standing on the substrate sheet. The covering sheet has a width substantially equal to the width of the diaper body, and is extending in a longitudinal direction from a front waist of the front flap. The covering sheet is a non-woven cloth foldable over an outer surface of the front flap, and serves as a female surface fastener tape.

A typical method to attach the paper diaper of this embodiment to the user's body is as follows. The user is laid down on a bed or a floor with its buttocks on the rear flap of the spread diaper. Then the front flap is put over his belly so as to wrap his crotch with the crotch. Then a pair of first wings extending laterally from the front flap are put round the opposite sides of the belly. Whereupon a pair of second wings extending laterally from the rear flap are put over the corresponding outer surfaces of the first wings. At the same time, the male surface fastener tapes are pressed against the outer surface of the non-woven cloth of the back sheet to bring the male engaging elements into engagement with the non-woven cloth. Then the covering sheet is folded over the front flap to cover the exposed surfaces of the male surface fastener tapes.

Thus attached to the user's body, the male surface fastener tapes engage with the surface of the front flap and/or the covering sheet. At this time, the covering sheet is curved along the user's waist and presses the non-woven cloth toward the user's belly via the male surface fastener tapes. Accordingly, even though the companion female surface fastener tape is a non-woven cloth which has only a limited engaging strength, the male surface fastener tapes engages the female surface fastener tape tightly. Further, while being under the above-mentioned pressure from outside, the male surface fastener tapes are also pulled away from each other, thus preventing the male surface fastener tapes from easily peeling off no mater how the infant moves.

Although preferably all of constituent parts of the diaper are made of biodegradable plastic, only a limited number of the constituent parts, e.g. the covering sheet and/or male surface fastener tape, of the diaper may be made of biodegradable plastic. Further, if the front flap and the covering sheet are each in the form of a structure of fibers, such as non-woven cloth, paper, or woven or knit cloth, it gives a comfortable touch or texture; economically, a non-woven cloth is preferable. Of course, one of the front flap and the covering sheet may be a synthetic resin sheet while the other is a non-woven cloth.

If the individual male surface fastener tape has a multiplicity of engaging elements on both front and rear surfaces, either the outer surface of the front flap or the covering sheet is preferably a non-woven cloth. With this structure, it is possible to fold and fasten the used diaper stably in compact size as waste. The method of folding and fastening the paper diaper is as follows. Firstly, the rear flap of the spread diaper body is folded inwardly so as to cover the entire area of the crotch, and at the same time, the first wings of the front flap are folded inwardly. Then the diaper body is wound in roll toward the front flap with the folded edge of the rear flap as a core. This winding is continued until the roll comes to the front waist of the front flap. The opposite male surface fastener tapes which are sticking out of the opposite ends of the roll of the diaper are then folded over the roll, and the corresponding engaging surfaces of the male surface fastener tapes are pressed against the front flap. Finally, the covering sheet (non-woven cloth) is folded over and pressed against the exposed surfaces of male surface fastener tapes to engage the front flap and/or the covering sheet, thus holding the used diaper in a compactly and stably folded posture.

Of course, each male surface fastener tape may have a multiplicity of male engaging elements on one of the front and rear surfaces. Further, although the substrate sheet and male engaging elements may be made of conventional fibers, but they are preferably molded of synthetic resin to realize a reduced cost.

### BRIEF DESCRIPTION OF THE DRAWINGS

Fig. 1 is a perspective view of a disposable paper diaper, in a spread posture before use, according to a typical embodiment of the present invention;
Fig. 2 is a perspective view showing the diaper attached to the user's body but fastened halfway;
Fig. 3 is a perspective view showing the diaper attached to the user's body and fastened completely;
Fig. 4 is an exploded perspective view of a disposable paper diaper, in a spread posture before use, according to another embodiment of the invention; and
Figs. 5A to 5F show the method in which the used diaper is folded and fastened as waste.

### DETAILED DESCRIPTION

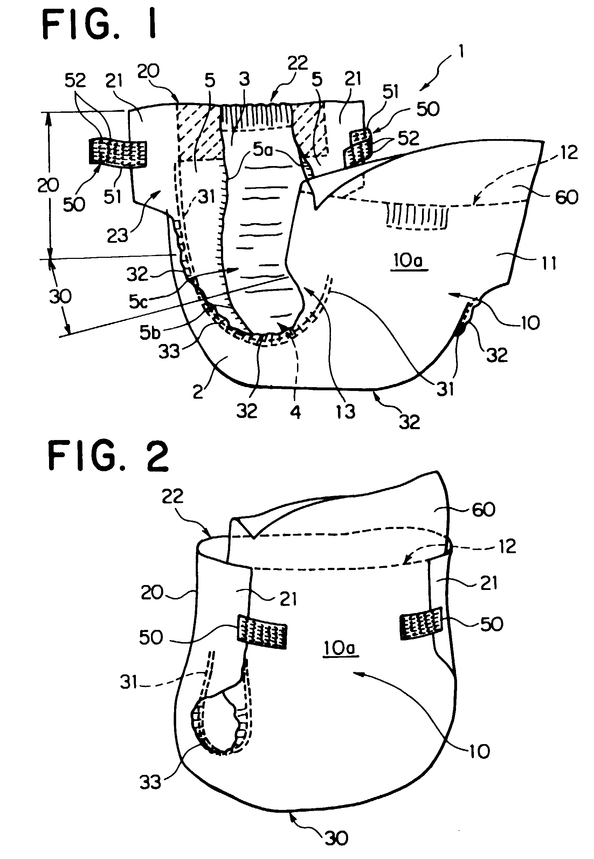

The different embodiments of the present invention will now be described in detail with reference to the accompanying drawings. Fig. 1 is a perspective view showing an infant-use disposable paper diaper, in an open posture before using, according to a typical embodiment of the invention, and Fig. 2 is a perspective view showing the paper diaper of Fig. 1 when attached to an infant's body but fastened halfway.

The present invention should by no means be limited to the infant-use disposable diaper and may be applied also to the adult-use disposable diaper with the same result. As a characteristic feature of the invention, in the infant-use paper diaper, a covering sheet 60 is folded over a front flap 10 of a diaper body 1 and is fastened by means of a surface fastener (described below) as shown in Fig. 2, the surface fastener cannot easily be unfastened. Particularly, once the diaper has thus been attached to the infant's body, the covering sheet 60 is curved along his waist to press the fastened portions to assist in securing an adequate degree of engaging strength. Thus the surface fastener does not easily peeled off.

The diaper of the first embodiment, like the conventional diaper, basically comprises the diaper body 1 to be attached to the infant's body, a surface fastener for holding the diaper body 1 to the infant's body. The diaper body 1 has the front flap 10, a rear flap 20 and a crotch 30 extending between the front and rear flaps 10, 20. The entire diaper body 1 is composed of a back sheet 2 to be located on the outer side remote from the skin of the infant's body when the diaper body 1 is attached, a top sheet 3 to be located on the side of skin of the infant's body when the diaper body 1 is attached to the infant's body, and a liquid absorbing pad 4 sandwiched between the back and top sheets 2, 3.

The back and top sheets 2, 3 are substantially identical in entire shape, each having a generally horizontal H shape with a pair of first/second wings 11, 21 extending in opposite directions from each end of a central rectangle when the back and top sheets 2, 3 are joined together in laminate. The non-illustrated liquid absorbing pad 4 is held between the back and top sheets 2, 3 and extends through their entire area except the peripheral edges and the first and second wings 11, 21. The peripheral edges of the back and top sheets 2, 3 are joined together by a suitable means such as adhesive or high-frequency welding. In this embodiment, each of the front and rear flaps 10, 20 has centrally in its waist 12, 22 a thin stretchable sheet incorporated therein to make the waist 12, 22 stretchable. Also, a plurality of stretchable yarns 31 are incorporated in and along each of opposite side edges 33 of the crotch 30 to form gathers 32.

Further, in this embodiment, a pair of leak-barrier tapes 5 are fixedly attached to opposite sides of the front flap 10, rear flap 20 and crotch 30 inwardly along their respective opposite side edges 13, 23, 33 by a suitable means such as adhesive or fusion under heat in such a manner that the outer edge of each leak-barrier tape 5 extends substantially in a straight line and is fixed while the outer edge is left open. A stretchable yarn 5a is fixedly attached to the open inner edge of the each leak-barrier tape 5 to form gathers 5b. Thus an excrement-leak-prevention hollow 5c is defined centrally in a region extending from the front flap 10 to the rear flap 20 on the side of the top sheet 3.

The back sheet 2 must have water-resistance and ventilation and, at the same time, as an important feature of the present invention, at least part of the back sheet 2 corresponding to the front flap 10 serves as a female surface fastener tape.

In the embodiment of Fig. 1, it is preferable to compose the back sheet 2 of at least two layers in order to secure these two features. In such event, a non-woven cloth 10a is laminated over a synthetic resin film as of polyethylene or polyolefin. Preferably they are joined together at a multiplicity of spots or lines by a suitable joining means such as adhesive, high-frequency fusion or thermal fusion. The resulting back sheet 2 is disposed in such a way that the non-woven cloth is on the outer side opposite to the skin of the infant's body when the diaper is attached.

From the ecological point of view, the substance for the disposable diaper of the present invention is preferably biodegradable plastic. This plastic is exemplified by microorganism-fermented resin such as a copolymer of hydroxybutanoic acid and hydroxyvaleric acid, natural polymer (starch) resin such as a blend of starch and metaphoric polyvinyl alcohol or a blend of starch and biodegradable synthetic polymer, fatty acid polyester, chemosynthetic resin such as polycaprolacton, and polylactic acid.

The top sheet 3 may be made of hydrophilic or hydrophobic material; however, since the top sheet 3 must allow liquid to pass and from an economical point of view it is preferable to use a non-woven cloth of synthetic fibers. The material of the synthetic fibers may be polyethylene, polypropylene, polyester and nylon, and more preferably the above-mentioned biodegradable plastic. Further, the top sheet 3 may be elastic; for example, such a non-woven cloth is produced by simultaneously spinning two kinds of synthetic resins different in contraction with hot water as composite fibers and then by treating the result composite fibers with hot water. During this production, the composite fibers and a predetermined quantity of synthetic resin powder having a melting point lower than that of the fibers are uniformly mixed to form a web. Then, the web is heated at a temperature higher than the melting point of the synthetic resin powder and lower than that of the fibers to melt the synthetic resin powder and, at the same time, the molten synthetic resin of the powder is collected at intersections of the fibers by utilizing a capillary phenomenon. Whereupon the molten synthetic resin is cooled to join the fibers at their intersections, thus completing the production of the non-woven cloth. As a simpler alternative, a predetermined quantity of adhesive may be uniformly sprayed over the fiber web to obtain a desired non-woven cloth. In this case, a suitable quantity of solvent is added to the adhesive.

The liquid absorbing pad 4 may be felt of cellulose fibers or hydrophilic synthetic fibers as of polyvinyl alcohol. Alternatively, liquid-absorbent gel grains or fibers may be mixed in such felt. The liquid-absorbent gel is exemplified by an inorganic substance such as silica gel and an organic composition such as cross-link polymer. Further, in order to hold liquid and also to retain the liquid-absorbent substance inside, the liquid-absorbing pad is preferably a laminate form composed of a plurality of thin-film fiber webs. Alternatively, as disclosed in the above-mentioned prior art publication, the liquid-absorbing pad 4 may include elastic fibers or thermally shrinkable fibers attached in stretched form to a fiber web, in which the intersections of fibers are joined so as to have a predetermined porosity, and may be stretchable.

The leak-barrier tapes 5 may be made of the same substance as that of the top sheet 3. And the stretchable yarns 5a fixedly attached in a straight line to the individual leak-barrier tape 5 along its open edge are elastic yarns as of natural rubber, synthetic rubber or elastomer resin, or crimped fiber yarns. Alternatively, the leak-barrier tape 5 may be stretchable through its entire area; in such event, a desired stretchability can be imparted to the non-woven cloth by incorporating the elastic or stretchable fibers of rubber and other various fibers of elastomer resin into the cloth.

In addition to the foregoing fundamental feature, the disposable diaper of the present invention has a primary characteristic feature as follows.

Yet referring to FIG. 1, at least a part of back sheet 2 corresponding to the outer surface of the front flap 10 is made of non-woven-cloth 10a and a pair of male surface fastener tapes 50 of the surface fastener are attached one to each of the two second wings 21, which extend laterally in opposite directions from opposite ends of a rear waist 22 of the rear flap 20. And a covering sheet 60, which has a width substantially equal to that of the front flap 10, is extending in a longitudinal direction from the front waist 12 of the front flap 10. The covering sheet 60 is folded over the front flap 10 so as to cover substantially the entire area of the front flap 10 where the male surface fastener tapes 50 are in engagement.

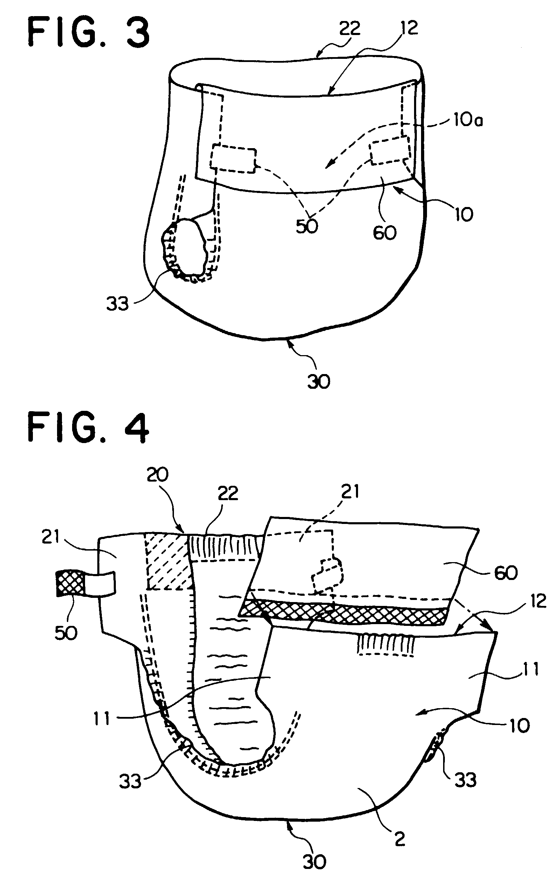

In the illustrated embodiment, the male surface fastener tapes 50 is a molded male surface fastener member in which a plurality of hook-shaped male engaging elements 52 are molded of a thermoplastic synthetic resin on a substrate sheet 51 of the same resin. If an economical view point is ignored, the molded male surface fastener member may be substituted by a woven or knit male surface fastener member whose male engaging elements 52 are in the form of monofilaments woven or knitted in a woven or knit cloth of fibers. In the molded male surface fastener member 50, the male engaging elements 52 should by no means be limited to the hook shape and may be mushroom-shaped. Further, the male engaging members 52 may be formed on opposite surfaces of the substrate sheet 51. Alternatively, the hook-shaped male engaging elements 52 may be molded on the front surface of the substrate sheet 51, and at the same time non-illustrated mushroom-shaped male engaging elements may be molded on the rear surface of the same substrate sheet 51. This alternative is more preferable because the male engaging elements 52 of the male surface fastener tapes 50 are brought into engagement with the non-woven-cloth 10a of the back sheet 2 and also with the non-woven-cloth covering sheet 60 when the covering sheet 60 is folded over the male surface fastener tapes 50 as shown in Fig. 3.

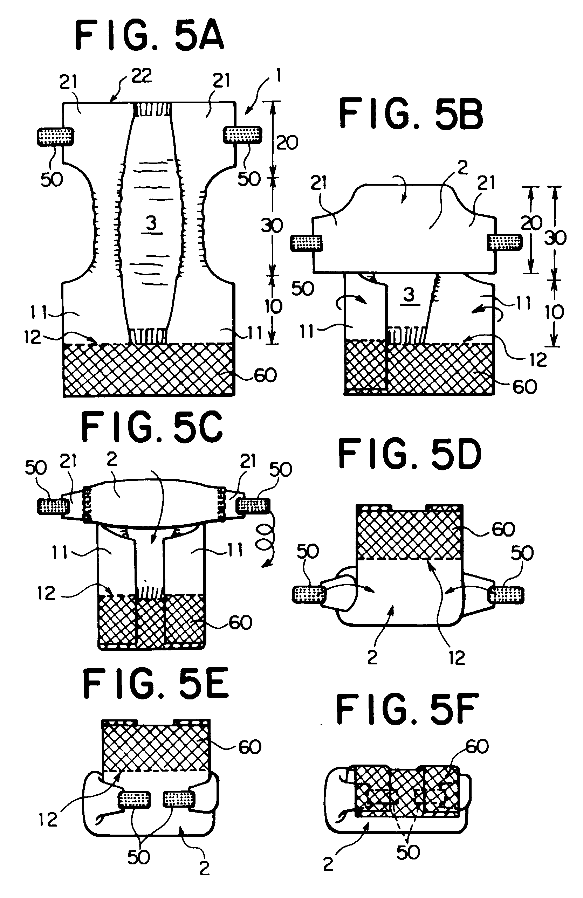

The method to attach the paper diaper of this embodiment to the infant's body will now be described. The infant is laid down on a bed or a floor with its buttocks on the rear flap 20 of the spread diaper, and then the front flap 10 is put over his belly so as to wrap his crotch with the crotch 30. Then the first wings 11 of the front flap 10 are put round the opposite sides of the belly. Whereupon the second wings 21 of the rear flap 20 are put over the corresponding outer surfaces of the first wings 11. And, at the same time, as shown in FIG. 2, the male surface fastener tapes 50 are pressed against the outer surface of the non-woven cloth 10a of the back sheet 2 to bring the male engaging elements 52 into engagement with the non-woven cloth 10a. Then the covering sheet 60 is folded over the front flap 10 to cover the exposed surfaces of the male surface fastener tapes 50 as shown in FIG.5.

Thus attached to the user's body, the male surface fastener tapes 50 engage with the surface of the front flap 10 and/or the covering sheet 60. At this time, the covering sheet 60 is curved along the infant's waist and presses the non-woven cloth toward the infant's belly via the male surface fastener tapes 50. Accordingly, even though the companion female surface fastener tape is a non-woven cloth which has only a limited engaging strength, the male surface fastener tapes 50 engages the female surface fastener tape tightly. Further, while being under the above-mentioned pressure from outside, the male surface fastener tapes 50 are also pulled away from each other, thus preventing the male surface fastener tapes 50 from easily peeling off no mater how the infant moves.

Namely, the engaging strength between the male surface fastener tapes 50 and the non-woven cloth 60 is small compared to that between the male surface fastener tapes 50 and an ordinary woven or knit pile web of fibers. Therefore, it is difficult for conventional paper diapers, which would tend to receive forces from various directions, to adapt a non-woven-cloth as a female surface fastener member. Whereas according to the present invention, even though the female surface fastener member is a non-woven-cloth, partly because of the engaging force between the male surface fastener tapes 50 and the non-woven cloth 10a, and partly because of the pressure that the covering sheet 60 put on the male surface fastener tapes 50 and the non-woven cloth 10a while in use, it is possible to secure adequate engaging strength. On the other hand, the male surface fastener tapes 50, the covering sheet 60 and the non-woven cloth 10a can be peeled off individually in a simple manner while not in use.

Further, the above-mentioned features also facilitates the method to fold the diaper especially the one in which each male surface fastener tape 50 has a plurality of engaging elements 52 on both front and rear surfaces. Therefore, it is possible to carry out packaging of a newly manufactured paper diaper as a compact product for shipping and to fold and fasten the used diaper in compact size as waste. The procedure of a first method of folding and fastening the paper diaper will now be described in detail with reference to FIGS. 5A to 5E.

Firstly, the rear flap 20 of the diaper body 1 which is spread with the top sheet 3 directed upwardly as shown in FIG. 5A, is folded inwardly (downwardly in the FIG. 5A) so as to cover the entire area of the crotch 30 as shown in FIG. 5B. At the same time, the first wings 11 of the front flap 10 are folded inwardly. Then the diaper body 1 is would in roll toward the front flap 10 with the folded edge of the rear flap 20 as a core as shown in Fig. 5C. This winding is continued until the roll comes to the front waist 12 of the front flap 10 as shown in FIG. 5D. At that time, the two male surface fastener tapes 50 each having on its front surface a multiplicity of male engaging elements 52 are sticking out laterally in opposite directions from opposite ends of the roll, and the non-woven cloth 10a of the front flop 10 is exposed to the peripheral surface of the roll.

Then, the opposite male surface fastener tapes 50 are folded inwardly over the rolled diaper body 1 as indicated by arrows in Fig. 5D and are then pressed against the exposed non-woven cloth 10a of the front flap 10 as shown in Fig. 5E. Thus the male engaging elements 52 of each male surface fastener member 50 is brought into engagement with the non-woven cloth 10a of the front flap 10. Finally, the covering sheet 60 is folded over the exposed surfaces of the male surface fastener tapes 50 and is pressed against them for fastening as shown in FIG.5F. As a result, the paper diaper has been folded and fastened in a compact roll as waste.

It depends on the number of windings of the roll whether the non-woven cloth of the covering sheet 60 engages with the front or rear surfaces of the male surface fastener tapes 50. Therefore, if each male surface fastener tape 50 has male engaging elements 52 only on one of its surfaces as the conventional diapers, the male surface fastener tapes 50 engage with only one of halves of the folded covering sheet 60. Whereas in this embodiment, since each male surface fastener tape 50 has a multiplicity of engaging elements 52 on opposite surfaces, it can be engaged with both the non-woven cloth 10a of the front flap 10 and the covering sheet 60 no matter how many times the diaper is wound. Since the male and female surface fastener tapes 50, 60 are thus fastened together face to face, it is particularly simple to perform such fastening, preventing the rolled diaper as waste from losing its roll shape.

It is troublesome from various point of views to handle the diaper after use. According to the above-described method of the present invention, the used paper diaper can be folded sanitarily into a compact roll as waste and can be fastened stably so as not to lose its roll shape. At that time, the number of windings of the diaper into a roll as shown in Fig. 5C depends on the volume of the excrement.

In the present invention, as mentioned in the above, since each male surface fastener tape 50 has a multiplicity of engaging elements 52 on opposite surfaces, it can be engaged with both the non-woven cloth 10a of the front flap 10 and the covering sheet 60 no matter how many times the diaper is wound. Since the male and female surface fastener tapes 50, 60 are thus fastened together face to face, it is particularly simple to perform such fastening, preventing the rolled diaper as waste from losing its roll shape. Further, even if part of the excrement happens to move over the leak-barrier tapes 5 of the crotch 30, the first and second wings 11, 21 shut the opposite ends of the diaper roll to prevent the excrement from leaking out of the diaper body 1, thus realizing the very sanitary and easy handling of the used diaper.

Fig. 4 is an exploded perspective view of an infant-use paper diaper according to another embodiment of the invention. The diaper of this embodiment is totally similar to that of the first embodiment except that the covering sheet 60 is a non-woven cloth separate from the back sheet 3 and adapted to be adhered to the upper edge of the waist 12 of the front flap 10 and that the back sheet 3 is a water-resistant and ventilative synthetic resin film. The substance of this synthetic resin film may be the same as that of the film described in connection with the first embodiment.

The method in which the paper diaper of the second embodiment is attached to the infant's body will now be described. The infant is laid down on a bed or a floor with its buttocks on the rear flap 20 of the spread diaper , and then the front flap 10 is put over his belly so as to wrap his crotch with the crotch 30. Then the first wings 11 of the front flap 10 are put round the opposite sides of the belly. Whereupon the second wings 21 of the rear flap 20 are put over the corresponding outer surfaces of the first wings 11 and, at the same time, the male surface fastener tapes 50 are pressed against the synthetic resin back sheet 2 of the front flap 10. Then the covering sheet 60 is folded and pressed over the front flap 10 to cover the exposed surfaces of the male surface fastener tapes 50 and to bring the male engaging elements 52 into engagement with the non-woven cloth 10a.

After the paper diaper has thus been attached to the infant's body, just as the first embodiment of the present invention, the male surface fastener tapes 50 engage with the surface of the front flap 10 and/or the covering sheet 60. At this time, the covering sheet 60 is curved along the infant's waist and presses the non-woven cloth toward the infant's belly via the male surface fastener tapes 50. Accordingly, even though the companion female surface fastener tape is a non-woven cloth which has only a limited engaging strength, the male surface fastener tapes 50 engages the female surface fastener tape tightly. Further, while being under the above-mentioned pressure from outside, the male surface fastener tapes 50 are also pulled away from each other, thus preventing the male surface fastener tapes 50 from easily peeling off no mater how the infant moves. The folding method of this embodiment is also identical to that of the first embodiment.

It is thus apparent that the present invention should by no means be limited to the foregoing embodiments and various modifications and changes may be suggested without departing from the scope and spirit of the invention.

As is apparent from the foregoing description, according to the disposable paper diaper of the present invention, partly since at least a part of the front flap 10 or the covering sheet 60 extending from the front waist 12 of the front flap 10 is a non-woven cloth 10a serving as the female surface tape, and partly since the covering sheet 60 is foldable, it is possible to cover the of the male surface fastener tapes 50 completely with the covering sheet 60. Further, partly since the folded covering sheet 60 assumes a curved posture fit to the curve of the belly of the user when attached to his body, partly since the covering sheet 60 engages the male surface fastener tapes 50 or presses the male surface fastener tapes 50 against the non-woven cloth 10a of the front flap 10, and partly since external forces would be exerted on the male surface fastener tapes 50 laterally in opposite directions, it is possible to secure an adequate engaging strength even though the female surface fastener tape is a non-woven cloth.

Also, since part of the front flap 10 and/or the covering sheet 60 is made of non-woven cloth serving as the female surface fastener tape, it is possible to unfasten the male and female surface fastener tapes with a low zip sound so that the used diaper can be exchanged with a new one without disturbing the infant in bed.

According to another alternative form, partly since each male surface fastener tape 50 has a multiplicity of male engaging elements 52 on both front and rear surfaces and partly since the covering sheet 60 is a non-woven cloth, it is possible not only to facilitate fastening and unfastening of the paper diaper but to guarantee adequate engaging strength. Thus a disposable paper diaper having a comfortable touch and foldable in a compact size has been realized.

## Claims

1. A disposable diaper comprising:
a diaper body (1) which has a front flap (10), a rear flap (20) and a crotch (30), and a pair of male surface fastener tapes; characterized in
that at least a part of outer surface of said front flap (10) is defined by a non-woven cloth (10a) serving as a female surface fastener tape;
that said pair of male surface fastener tapes (50) are extending laterally in opposite directions from said rear flap (20) and that each of said male surface fastener tapes (50) is composed of a substrate sheet (51) and a multiplicity of male engaging elements (52) standing on said substrate sheet (51); and
that a covering sheet (60) having a width substantially equal to the width of said diaper body (1) is extending in a longitudinal direction from a front waist (12) of said front flap (10), and that said covering sheet (60) is foldable over said non-woven cloth (10a).

2. A disposable diaper according to claim 1, characterized in that said covering sheet (60) is a fiber structure of non-woven, woven or knit cloth or paper.

3. A disposable diaper according to claim 1, characterized in that said covering sheet (60) is a synthetic resin sheet.

4. A disposable diaper comprising:
a diaper body (1) which has a front flap (10), a rear flap (20) and a crotch (30), and a pair of male surface fastener tapes; characterized in
that said pair of male surface fastener tapes (50) are extending laterally in opposite directions from said rear flap (20), and that each of said male surface fastener tapes (50) is composed of a substrate sheet (51) and a multiplicity of male engaging elements (52) standing on said substrate sheet (51);
that a covering sheet (60) having a width substantially equal to the width of said diaper body (1) is extending in a longitudinal direction from a front waist (12) of said front flap (10), and that said covering sheet (60) is a non-woven cloth foldable over an outer surface of said front flap (10) and serving as a female surface fastener tape.

5. A disposable diaper according to claim 1 or 4, characterized in that said male engaging elements (52) of each of said male surface fastener tapes (50) stand on both front and rear surfaces of said substrate sheet (51) for engagement with both said front flap (10) and said covering sheet (60).

6. A disposable diaper according to claim 1 or 4, characterized in that said male engaging elements (52) of each of said male surface fastener tapes (50) stand on one of front and rear surfaces of said substrate sheet (51), and that a corresponding one of said front flap (10) and said covering sheet (60) is a non-woven cloth engageable with said male surface fastener tapes (50).

7. A disposable diaper according to claim 1 or 4, characterized in that all of constituent parts of said diaper are made of biodegradable plastic.

8. A disposable diaper according to claim 1 or 4, characterized in that a limited number of constituent parts of said diaper is made of biodegradable plastic.

9. A disposable diaper according to claim 7 or 8, characterized in that said substrate sheet (51) and said male engaging elements (52) are integrally molded of synthetic resin.
